# EUROPÄISCHE PATENTSCHRIFT

(11) **EP 1 870 134 B1**
(45) Veröffentlichungstag und Bekanntmachung des Hinweises auf die Patenterteilung: **21.11.2012**
(21) Anmeldenummer: 07005716.1
(22) Anmeldetag: 20.03.2007
(51) Int. Cl.: A61N 5/10

(54) **Vorrichtung zur Überwachung der Ausrichtung von Markierungslasern**
Device for monitoring the alignment of marking lasers
Dispositif de surveillance de l'alignement de lasers de marquage

(30) Priorität: 19.06.2006 DE 102006028053
(43) Veröffentlichungstag der Anmeldung: 26.12.2007
(73) Patentinhaber: LAP GmbH Laser Applikationen, D-21337 Lüneburg (DE)
(72) Erfinder: Röckseisen, Armin, Dr., 21379 Scharnebeck (DE)
(74) Vertreter: Hauck Patent- und Rechtsanwälte

(56) Entgegenhaltungen:
- EP-A2- 1 211 480
- WO-A-2006/055770
- WO-A2-2006/017013
- WO-A2-2007/124902
- DE-A1- 4 418 216
- FR-A1- 2 721 497
- GB-A- 2 054 142
- GB-A- 2 331 360
- US-A- 5 142 559
- US-A- 6 041 249
- US-A- 6 099 522
- US-A1- 2005 174 582
- US-B1- 6 308 428

## Beschreibung

Die vorliegende Erfindung betrifft eine Vorrichtung zur Überwachung von Markierungslasern in Räumen zur Diagnose und/oder Behandlung bei der Strahlentherapie.

Eine Vorrichtung zur Ausrichtung von Lasern ist aus US 6 308 428 B1 bekannt.

In der Strahlentherapie werden verschiedene bildgebende, diagnostische Verfahren eingesetzt, die Aufschluß über eine eventuelle Tumorerkrankung sowie über Größe, Form und Lage des Tumors geben. Um zu einem späteren Zeitpunkt der Bestrahlung, die Lage des Tumors erfolgreich an einem Therapiegerät wieder auffinden zu können, ist es erforderlich, die räumliche Information der diagnostischen Daten auf das Therapiegerät zu übertragen. Zur Übertragung der Daten ist es ein gebräuchliches Verfahren, das bei der diagnostischen Bildgebung und der anschließenden Bestrahlungsplanung ermittelte Isozentrum, welches zugleich das Zentrum des Tumors darstellt, durch die Projektion von Laserlinien anzuzeigen und am Körper des Patienten zu markieren. Mit Hilfe dieser Markierung auf dem Patientenkörper kann der Patient zu einem späteren Zeitpunkt erneut wieder ausgerichtet werden. Erfolgte die Markierung beispielsweise bei einem bildgebenden, diagnostischen Verfahren, so kann der Patient im Therapieraum relativ zu fest installierten Lasern, die auf das Isozentrum der Therapiemaschine ausgerichtet und fein justiert sind, erneut ausgerichtet werden. Um eine hohe Qualität bei der Ausrichtung sicherstellen zu können, ist es erforderlich die Ausrichtung insbesondere die Feinausrichtung der Laser zu überprüfen.

Der Erfindung liegt die Aufgabe zugrunde, eine Vorrichtung bereitzustellen, die es erlaubt, mit einfachen Mitteln die korrekte Ausrichtung der Markierungslaser zu überprüfen und etwaige Abweichungen frühzeitig anzuzeigen.

Erfindungsgemäß wird die Aufgabe durch eine Vorrichtung mit den Merkmalen aus Anspruch 1 gelöst. Vorteilhafte Ausgestaltung bilden die Gegenstände der Unteransprüche.

Die erfindungsgemäße Vorrichtung dient zur Überwachung der Ausrichtung von Markierungslasern in Räumen zur Diagnose und/oder Behandlung bei der Strahlentherapie. Die Überwachungsvorrichtung ist gekennzeichnet durch ein Gehäuse, das mit Haltemitteln zur Montage an der Wand in dem Raum versehen ist und das einen Fotosensor sowie eine Auswerteeinheit aufweist. Der Fotosensor ist lichtempfindlich und zeigt die Position, in der das Licht auf den Fotosensor fällt an.

Die Auswerteeinheit mißt die Position des von dem Laser erzeugten Lichts und vergleicht diese mit einer vorgegebenen Referenzposition. Aus der Abweichung von der Referenzposition, und bevorzugt bei Überschreiten einer maximal zulässigen Abweichung von der Referenzposition, erzeugt die Auswerteeinheit ein entsprechendes Warnsignal, das die fehlende Justierung anzeigt. Die erfindungsgemäße Vorrichtung erlaubt es, durch den Einsatz von Fotosensoren in dem Überwachungsgerät sehr genau die Richtigkeit der Justage der Markierungslaser zu prüfen.

In einer besonders bevorzugten Ausgestaltung bildet die Auswerteeinheit einen räumlichen Mittelwert über ein räumlich verteiltes Signal. Insbesondere bietet die Verwendung von einem zeilenförmigen Sensor den Vorteil, daß auch Laserlinien, die aufgrund der Strahldivergenz aufgeweitet sind, in ihrer Strahllage zuverlässig ausgewertet werden können, was einer beispielsweise rein optischen Sichtkontrolle deutlich überlegen ist.

In einer bevorzugten Ausgestaltung ist die Auswerteeinheit ferner über eine Schnittstelle mit einer zentralen Datenverarbeitungseinrichtung verbunden. Die Datenverarbeitungseinrichtung führt die Signale der einzelnen Auswerteeinheiten zusammen. So kann beispielsweise vor der Inbetriebnahme nicht nur die Position der Laser überprüft sondern auch aufgezeichnet werden, um später deren Ausrichtung nachvollziehen zu können.

In einer bevorzugten Ausgestaltung generiert die Auswerteeinheit Signale für eine Verstelleinrichtung zur Ausrichtung der Laser. Auf diese Weise kann zusätzlich zu dem Signal, das die Abweichung der Laser aus der Referenzposition anzeigt, ein Steuersignal generiert werden, um die Laser automatisch auszurichten. Auf diese Weise kann die Feinjustierung der Laser in regelmäßigen Abständen nachgestellt werden.

Die vorliegende Erfindung wird nachfolgend anhand eines Ausführungsbeispiels näher erläutert:
- Fig. 1: zeigt in einer schematischen Ansicht ein in einem Raum installiertes Überwachungsgerät,
- Fig. 2: zeigt das Gehäuse eines Überwachungsgerätes in einer perspektivischen Ansicht und
- Fig. 3: zeigt in einer Blockansicht die wesentlichen Komponenten im Hinblick auf die Überwachung und die Feinjustierung der Markierungslaser zeigt.

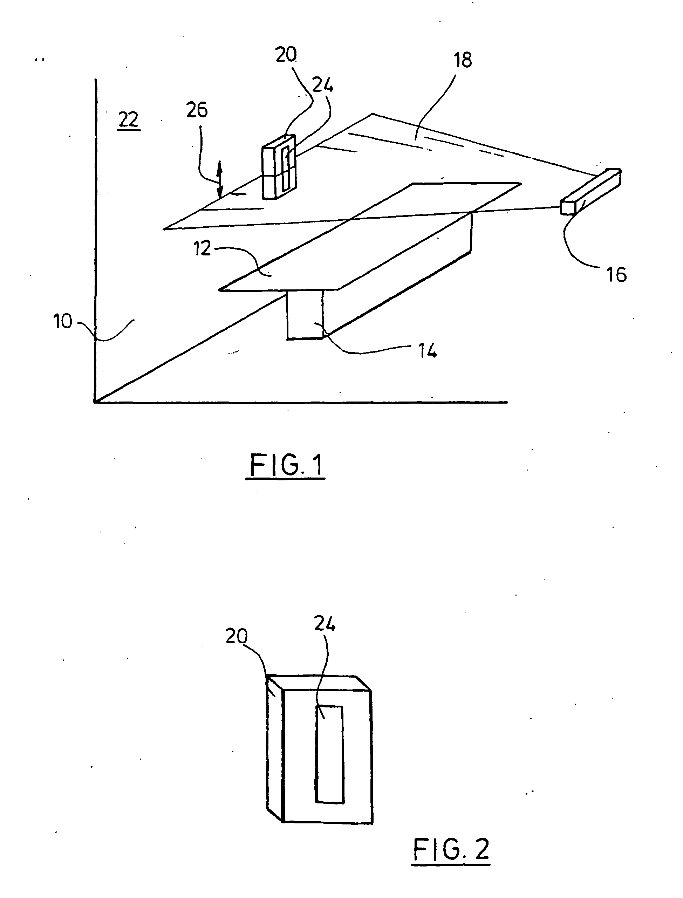

In Fig. 1 zeigt in einer schematischen Ansicht einen Raum 10 für eine Therapiemaschine. In dem Raum ist eine Patientenunterlage 12 vorgesehen, die mit Hilfe einer im Tischfuß 14 angeordneten Verstelleinrichtung in eine gewünschte Position relativ zu der Therapiemaschine (nicht dargestellt) verfahren werden kann. Ein auf der Unterlage liegender Patient wird durch einen seitlich angeordneten Laser bestrahlt. Der Laser erzeugt eine Lichtebene 18, die in dem dargestellten Beispiel ungefähr parallel zur Oberfläche der Unterlage 12 verläuft. Nicht dargestellt in Fig. 1 sind die weiteren Linienlaser, die zur Ausrichtung des Patienten eingesetzt werden. Üblicherweise ist auf Höhe des auf der Unterlage 12 liegenden Patienten seitlich ein Paar von Linienlasern angeordnet, deren Lichtebenen ungefähr parallel zur Oberfläche der Unterlage 12 verlaufen. Ferner ist ein Linienlaser zu einer Sagittallinie, entlang der Längsachse des Patienten, vorgesehen. Um möglichst genau dem Patienten ausrichten zu können, ist zusätzlich eine quer stehende Lichtebene auf den Patienten gerichtet.

Um die Feinjustierung des Lasers 16 zu prüfen, ist erfindungsgemäß ein Überwachungsgerät 20 vorgesehen, das in einer vorbestimmten Position an der Wand 22 in dem Behandlungsraum montiert ist. Das Überwachungsgerät 20 besitzt einen Zeilensensor 24 (vgl. Fig. 2). Der Zeilensensor 24 ist in der montierten Position quer zur Lichtebene 18 angeordnet. Eine Dejustierung des Lasers 16 entlang der Richtung 26 wird durch den Zeilensensor 24 erfaßt und kann hier ausgewertet werden.

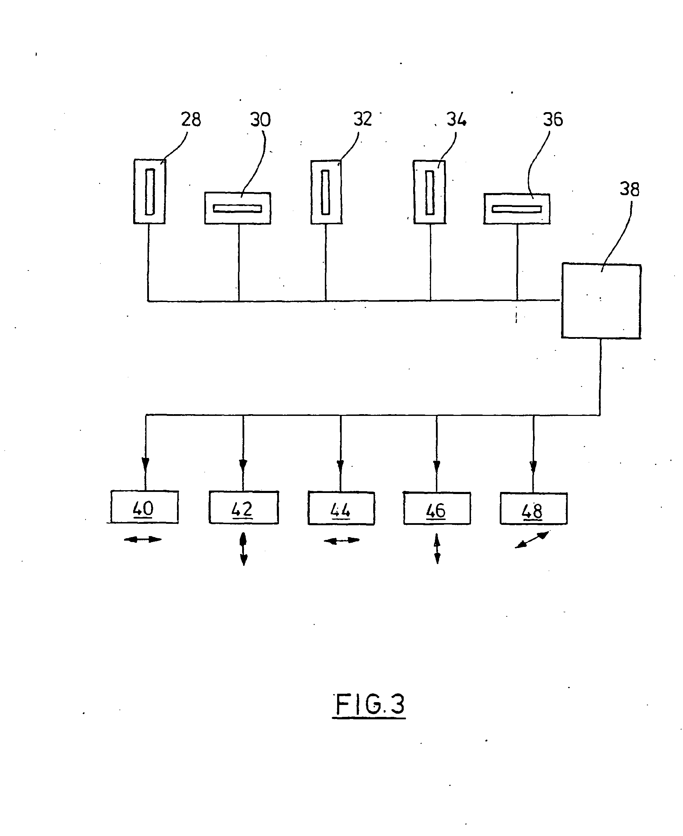

Fig. 3 zeigt eine schematische Ansicht einer installierten Überwachung für die Ausrichtung der Markierungslaser an. In dem dargestellten Beispiel sind fünf Überwachungsgeräte 28 bis 36 vorgesehen. Jedes der Überwachungsgeräte ermittelt die Position, in der der Laser auf das Überwachungsgerät fällt. Neben der lokalen Auswertung der Meßsignale dahingehend, ob die Lasersignale in der vorbestimmten Referenzposition auf das Überwachungsgerät treffen, werden die Positionssignale an einen zentralen Rechner 38 weitergeleitet, der die Positionssignale auswertet und entsprechende Steuersignale für einen Verstellantrieb der Laser generiert. In dem in Fig. 3 dargestellten Ausführungsbeispiel kann jeder Laser 40 bis 48 jeweils in einer Richtung vor- und zurückgefahren werden. Grundsätzlich könnte die Ansteuerung der Verstellantriebe auch direkt von jedem der Überwachungsgeräte erfolgen. Die Positionsdaten zunächst in einem zentralen Computer 38 zusammenzuführen besitzt den Vorteil, daß Verstellvorgänge zentral protokolliert werden können und so beispielsweise systematische Fehler leichter erkannt werden können.

Die Haltemittel für das Überwachungsgerät besitzen ebenfalls den Vorteil, daß diese als lösbare Haltemittel ausgebildet sein können, so daß beispielsweise während der Bestrahlung das erfindungsgemäße Gerät nicht bestrahlt wird.

## Patentansprüche

1. Vorrichtung zur Überwachung der Ausrichtung von Markierungslasern (16) in einem Raum (10) zur Diagnose und/oder Behandlung bei Strahlentherapie, umfassend
- ein Gehäuse, das Folgendes enthält:
- einen zeilenförmig sich erstreckenden Fotosensor (24) und eine Auswerteeinheit, die eingerichtet ist, die Position des von dem Laser (16) erzeugten Lichts (18) auf dem Fotosensor (24) mit einer vorgegebenen Referenzposition zu vergleichen und bei einer Abweichung der gemessenen Position von der Referenzposition ein entsprechendes Signal zu erzeugen,
- wobei die Auswerteeinheit eingerichtet ist, bei einem räumlich auf dem Sensor (24) verteilten Signal den räumlichen Mittelwert des Sensorsignals zu bilden
- **dadurch gekennzeichnet, dass** das Gehäuse mit Haltemitteln zur Montage an der Wand in dem Raum (10) versehen ist.

2. Vorrichtung nach Anspruch 1, **dadurch gekennzeichnet, dass** die Auswerteeinrichtung über eine Schnittstelle mit einer zentralen Datenverarbeitung (38) verbindbar ist.

3. Vorrichtung nach einem der Ansprüche 1 oder 2, **dadurch gekennzeichnet, dass** die Auswerteeinheit eingerichtet ist, Signale für eine Verstelleinrichtung zur Ausrichtung der Laser (16) zu generieren.

## Claims

1. Apparatus for monitoring the alignment of marking lasers (16) in a room (10) for diagnosis and/or treatment in radiation therapy, comprising
- a housing, which contains the following:
- a linearly extending photosensor (24) and an analysing unit, which is designed to compare the position of the light (18) generated by the laser (16) on the photosensor (24) with a predetermined reference position, and to generate a corresponding signal upon a deviation of the measured position from the reference position, wherein the analysing unit is designed to form the spatial mean value of the sensor signal when the signal is spatially distributed on the sensor (24), **characterized in that** the housing is provided with holding means for the installation on the wall in the room (10).

2. Apparatus according to claim 1, **characterised in that** the analysing unit is connectable to a central data processing unit (38) via an interface.

3. Apparatus according to claim 1 or 2, **characterised in that** the analysing unit is designed to generate signals for an adjustment device for the alignment of the lasers (16).

## Revendications

1. Dispositif de surveillance de l'alignement de lasers de marquage (16) dans une pièce (10) prévue pour le diagnostic et/ou le traitement en radiothérapie, comprenant
- un boîtier, qui contient les éléments suivants :
- un photocapteur (24) se projetant en lignes et une unité d'évaluation qui est agencée pour comparer la position de la lumière (18) produite par le laser (16) sur le photocapteur (24) à une position de référence spécifiée, et en cas de divergence de la position mesurée par rapport à la position de référence, pour produire un signal correspondant,
l'unité d'évaluation étant disposée pour former, lorsqu'un signal est réparti spatialement sur le capteur (24), la valeur médiane spatiale du signal de capteur,
**caractérisé en ce que** le boîtier est muni de moyens de fixation pour le montage mural dans la pièce (10).

2. Dispositif selon la revendication 1, **caractérisé en ce que** l'unité d'évaluation peut être reliée par une interface à un traitement de données central (38).

3. Dispositif selon une des revendications 1 ou 2, **caractérisé en ce que** l'unité d'évaluation est agencée pour générer des signaux pour un dispositif de réglage pour l'alignement du laser (16).
